# EUROPÄISCHE PATENTANMELDUNG

(11) **EP 0 940 153 A1**
(43) Veröffentlichungstag der Anmeldung: **08.09.1999**
(21) Anmeldenummer: 99103851.4
(22) Anmeldetag: 27.02.1999
(51) Int. Cl.: A61M 5/32

(54) **Nadelschutz für den Nadelteil eines Spritzenkörpers**

(30) Priorität: 02.03.1998 DE 19808752
(71) Anmelder: Bünder Glas GmbH, 32257 Bünde (DE)
(72) Erfinder: Amann, Wolf-Rüdiger, 32429 Minden (DE); Stohlmann, Erhard, 32257 Bünde (DE)
(74) Vertreter: Schirmer, Siegfried, Dipl.-Ing.

(57) **Zusammenfassung**

Der erfindungsgemäße Nadelschutz (3) ermöglicht eine ausreichende Sicherheit gegen Verletzung durch die Nadelspitze sowohl im Gebrauchszustand als auch auf dem Entsorgungsweg.

Hierzu dient eine auf dem Spritzenkörper (2) in Längsrichtung verschiebbare Sicherheitsschutzkappe (3), die überwiegend aus elastischem Material gebildet ist. Diese Sicherheitsschutzkappe (3) weist auf der Innenseite eine obere (4) und mindestens eine untere (402) Raststellung auf, denen auf dem Spritzenkörper (2) entsprechend angepaßte Aufnahmen (6) zugeordnet sind. Zweckmäßigerweise weist die Sicherheitsschutzkappe (3) eine solche Länge auf, daß in einer oberen Verrastungsstelle die Nadel (5) abgedeckt ist.

## Beschreibung

Die Erfindung betrifft einen Nadelschutz für den Nadelteil eines Spritzenkörpers in Form einer die Nadel abdeckenden Schutzkappe mit einer auf dem Spritzenkörper in Längsrichtung verschiebbare Sicherheitsschutzkappe.

In der EP 0 734 738 ist eine Sicherheitsspritze mit einer Schutzhülse offenbart. Bei dieser bekannten Spritze ist die Schutzhülse in der zurückgezogenen Stellung durch Vorsprünge festgehalten, die an einem Kragen anstehen. Erst wenn die Schutzhülse auf dem Spritzenzylinder so verdreht wird, daß die Vorsprünge mit Ausnehmungen in dem Kragen fluchten, läßt sich die Schutzhülse zur Nadelspitze hin verschieben. In der Schutzstellung wird die Schutzhülse durch Rastzähne festgehalten, die mit einer Rastwand des Kragens in Eingriff kommen.

Der Erfindung liegt die Aufgabe zugrunde, einen Nadelschutz für eine Spritze so auszubilden, daß der Nadelschutz während der Verabreichung der Spritze sicher in der zurückgezogenen Stellung gehalten ist, daß nach Gebrauch der Spritze eine ausreichende Sicherheit gegen Verletzungen der Nadelspitze gegeben ist und daß der Nadelschutz einfach herzustellen ist.

Erfindungsgemäß wird diese Aufgabe durch einen Nadelschutz gelöst, der dadurch gekennzeichnet ist, daß die Sicherheitsschutzkappe auf der Innenseite eine obere und mindestens eine untere Resthaltetung aufweist und daß die Rasthalterungen als umlaufende Ringnut ausgebildet sind, denen als Aufnahme eine Ringwulst zugeordnet ist. Zweckmäßigerweise besteht die Sicherheitsschutzkappe ganz oder teilweise aus elastischem Material.

Die Ringwulst kann Bestandteil der Schutzkappe oder bei einer Variante auch als separates Teil auf den Spritzenkörper aufgesetzt sein. Es liegt im Wesen der Erfindung, daß die Sicherheitsschutzkappe eine solche Länge aufweist, daß in der oberen Verrastungsstellung die Nadel abgedeckt ist.

In weiterer Ausgestaltung der Erfindung ist auf die Sicherheitsschutzkappe ein verschiebbar gelagerter Halterungsring aufgeschoben, wobei der Halterungsring zwischen einer oberen und einer unteren umlaufenden Rille verschoben werden kann. Zweckmäßigerweise weist der Halterungsring Haltenocken auf, die als umlaufender Ring ausgebildet sein können. Es ist vorteilhaft, den Halterungsring mit den Haltenocken einstückig aus Kunststoff herzustellen.

Ein Ausführungsbeispiel der Erfindung ist in der Zeichnung dargestellt und wird nachfolgend näher beschrieben. Es zeigen jeweils in der Ansicht:

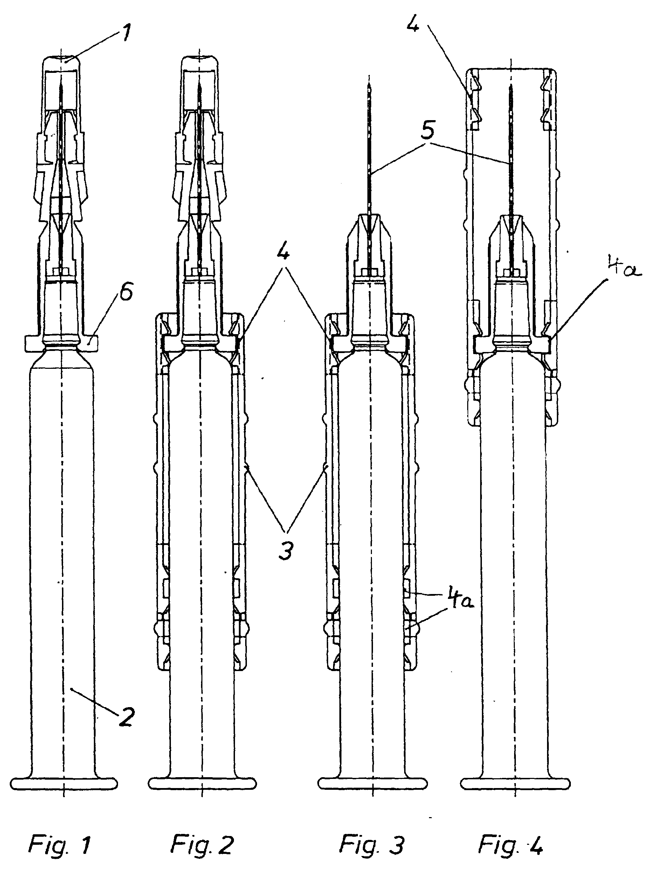

Fig. 1 einen Spritzenkörper mit Nadel und einer bekannten Schutzkappe;
Fig. 2 wie Fig. 1, jedoch mit aufgeschobener Sicherheitsschutzkappe;
Fig. 3 wie Fig. 2, jedoch im Benutzungszustand;
Fig. 4 wie Fig. 3, jedoch mit verschobener Sicherheitsschutzkappe;
Fig. 5 einen Spritzenkörper mit einem Zusatzring;
Fig. 6 einen Spritzenkörper mit Nadel- und Schutzkappe sowie aufgeschobener Sicherheitsschutzkappe mit Halterungsring in Ruhestellung und
Fig. 7 wie Fig. 6, jedoch mit versehobenem Halterungsring in Funktionsstellung.

Auf einen aus Glas oder Kunststoff bestehenden Spritzenkörper 2 ist zum Schutz der Nadel 5 eine Schutzkappe 1 aufgeschoben. Diese Schutzkappe 1 besitzt eine Ringwulst 6, auf die erfindungsgemäß die auf den Spritzenkörper 2 aufgeschobene Sicherheitsschutzkappe 3 mit einer Rasthalterung 4 aufgebracht ist. Für die Entsorgung des Spritzenkörpers 2 wird zum Schutz gegen Verletzungen durch die Nadel diese Sicherheitsschutzkappe 3 in Richtung der Nadel 5 verschoben, bis die Ringwulst 6 in eine der am oberen Ende der Sicherheitsschutzkappe 3 befindliche Rasthalterung 4a einführbar ist. Diese Einrastung setzt voraus, daß die Sicherheitsschutzkappe 3 zumindestens im Bereich der Rasthalterungen 4 und 4a elastische Eigenschaften aufweist. Zweckmäßigerweise ist jedoch die Sicherheitsschutzkappe 3 einstückig aus Kunststoff mit einer gewissen Eigenelastizität im Spritzgießverfahren hergestellt.

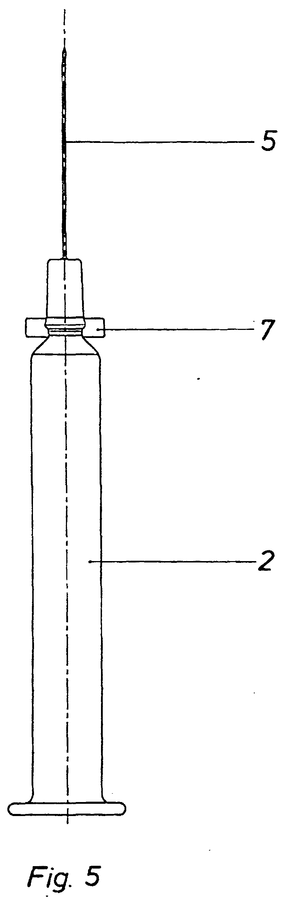

Figur 5 zeigt eine Variante der Verrastung der Sicherheitsschutzkappe 3. Besitzt die bekannte Schutzkappe I keine Ringwulst 6, so wird als Ersatz ein Zusatzring 7 auf den Spritzenkörper 2 aufgeschoben, der zum Einrasten in die Rasthalterungen 4; 4a ausgebildet ist.

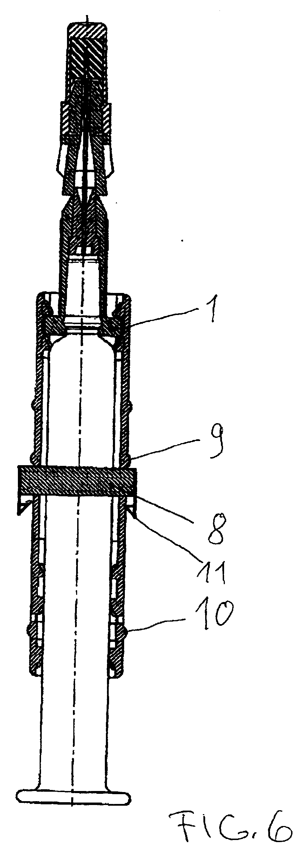

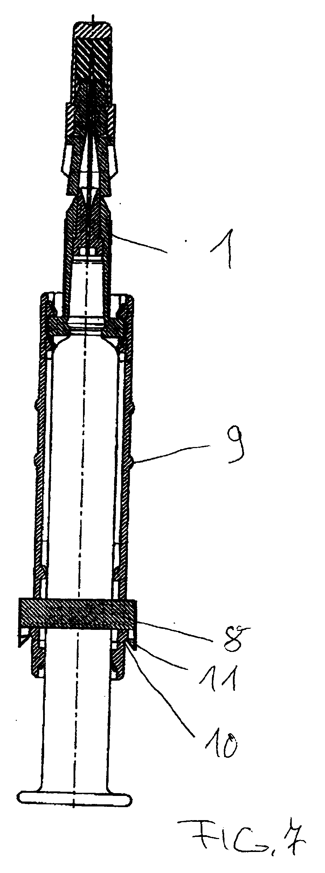

In den Figuren 6 und 7 ist eine Ausführung mit einem auf die Sicherheitsschutzkappe 3 aufgeschobenen Halterungsring 8 dargestellt. Dieser Halterungsring 8 ist auf der Sicherheitsschutzkappe 3 zwischen einer oberen umlaufenden Rille 9 und einer unteren umlaufenden Rille 10 verschiebbar. Der Halterungsring 8 weist Haltenocken 11 auf, wobei der Halterungsring 8 mit den Haltenocken 11 einstückig aus Kunststoff hergestellt ist.

Nach der Montage der Sicherheitsschutzkappe 3 wird der Halterungsring 8 aus der in Fig. 6 dargestellten Stellung in die in Fig. 7 dargestellte Stellung verschoben. In dieser Stellung ist eine Rastverbindung zwischen den Haltenocken 11 und der umlaufenden Rille 10 existent, wodurch gleichzeitig eine sichere Arretierung der Sicherheitsschutzkappe 3 am Spritzenkörper 2 hergestellt ist.

### Aufstellung der Bezugszeichen:

- 1: Schutzkappe
- 2: Spritzenkörper
- 3: Sicherheitsschutzkappe
- 4: Rasthalterung
- 4a: Rasthalterung
- 5: Nadel
- 6: Ringwulst
- 7: Zusatzring
- 8: Ring
- 9: obere Rille
- 10: untere Rille
- 11: Haltenocken

## Patentansprüche

1. Nadelschutz für den Nadelteil eines Spritzenkörpers in Form einer die Nadel abdeckenden Schutzkappe mit einer auf dem Spritzenkörper in Längsrichtung verschiebbare Sicherheitsschutzkappe, dadurch gekennzeichnet, daß die Sicherheitsschutzkappe (3) auf der Innenseite eine obere und mindestens eine untere Rasthalterung (4; 4a) aufweist und daß die Rasthalterungen (4; 4a) als umlaufende Ringnut ausgebildet sind, denen als Aufnahme eine Ringwulst (6) zugeordnet ist.

2. Nadelschutz nach Anspruch 1, dadurch gekennzeichnet, daß die Sicherheitsschutzkappe (3) ganz oder teilweise aus elastischem Material gebildet ist.

3. Nadelschutz nach Anspruch 1, dadurch gekennzeichnet, daß die Ringwulst (6) Bestandteil der Schutzkappe (1) ist.

4. Nadelschutz nach Anspruch 1, dadurch gekennzeichnet, daß zur Bildung einer Ringwulst (6) ein separates Teil in Form eines Zusatzrings (7) auf den Spritzenkörper (2) aufschiebbar ist.

5. Nadelschutz nach einem der Ansprüche 1 bis 4, dadurch gekennzeichnet, daß die Sicherheitsschutzkappe (3) eine solche Lange aufweist, daß in der oberen Verrastungsstellung die Nadel (5) abgedeckt ist.

6. Nadelschutz nach einem der Ansprüche 1 bis 5, gekennzeichnet durch einen auf die Sicherheitsschutzkappe (3) verschiebbar gelagerten Halterungsring (8).

7. Nadelschutz nach Anspruch 6, dadurch gekennzeichnet, daß der Halterungsring (8) zwischen einer oberen und einer unteren umlaufenden Rille (9; 10) verschiebbar ist.

8. Nadelschutz nach Anspruch 6 oder 7, dadurch gekennzeichnet, daß der Halterungsring (8) Haltenocken (11) aufweist.

9. Nadelschutz nach Anspruch 8, dadurch gekennzeichnet, daß die Haltenocken (11) als umlaufender Ring ausgebildet sind.

10. Nadelschutz nach einem der Ansprüche 6 bis 9 dadurch gekennzeichnet, daß der Halterungsring (8) mit den Haltenocken (11) einstückig aus Kunststoff hergestellt ist.
